# EUROPÄISCHE PATENTSCHRIFT

(11) **EP 4 025 818 B1**
(45) Veröffentlichungstag und Bekanntmachung des Hinweises auf die Patenterteilung: **16.04.2025**
(21) Anmeldenummer: 19768733.8
(22) Anmeldetag: 03.09.2019
(51) Int. Cl.: F16L 37/252, F16L 37/248, F16L 37/24, A61M 39/10

(54) **VERFAHREN ZUR VERBINDUNG VON ABSAUGSCHLÄUCHEN FÜR KÖRPERFLÜSSIGKEITEN MIT EINEM BEHÄLTER UND SCHLAUCH-SCHNELLVERBINDER FÜR ABSAUGSCHLÄUCHE FÜR KÖRPERFLÜSSIGKEITEN**
METHOD FOR COUPLING BODY-FLUID SUCTION TUBES TO A CONTAINER, AND QUICK-RELEASE COUPLING FOR BODY-FLUID SUCTION TUBES
PROCÉDÉ DE RACCORDEMENT DE TUYAUX D'ASPIRATION POUR LIQUIDES CORPORELS À UN RECIPIENT, ET RACCORDEMENT RAPIDE DE TUYAUX POUR TUYAUX D'ASPIRATION POUR LIQUIDES CORPORELS

(43) Veröffentlichungstag der Anmeldung: 13.07.2022
(73) Patentinhaber: ATMOS MedizinTechnik GmbH & Co. KG, 79853 Lenzkirch (DE)
(72) Erfinder: ECKERT, Patrick, 79809 Weilheim Remetschwiel (DE); WEINMANN, Theron, 79777 Ühlingen-Birkendorf (DE); KAISER, Ralf, 79780 Stühlingen (DE); WILLE, Michael Hans, 79822 Titisee-Neustadt (DE)
(74) Vertreter: Westphal, Mussgnug & Partner Patentanwälte mbB
(86) Internationale Anmeldenummer: PCT/EP2019/073424
(87) Internationale Veröffentlichungsnummer: WO 2019/234261

(56) Entgegenhaltungen:
- EP-A1- 0 633 039
- DE-A1- 102008 046 098
- US-A- 2 525 879

## Beschreibung

Die Erfindung betrifft ein Verfahren zur Verbindung von Absaugschläuchen für Körperflüssigkeiten mit einem Behälter, und einen Schlauch-Schnellverbinder für Absaugschläuche für Körperflüssigkeiten.

Auch wenn beim Transport von Flüssigkeiten durch Leitungen und Schläuche in einen Behälter das Herstellen einer dichten Verbindung stets eine wichtige Anforderung darstellt, treten beim Management von abgesaugten Körperflüssigkeiten besondere Anforderungen hinzu. Erstens sind die Anforderungen an die Dichtigkeit tendenziell erhöht, da es sich einerseits regelmäßig um unter Aspekten der Hygiene problematische Flüssigkeiten handelt und andererseits die Effektivität der Absaugung nicht durch Lecks reduziert werden darf. Zweitens muss das Herstellen der Verbindung einfach, schnell und zuverlässig erfolgen, damit es entweder vom Patienten, der in vielen Fällen alt oder gebrechlich ist, selbst durchgeführt werden kann oder, wenn es von Pflegepersonal durchgeführt wird, keine zusätzliche Belastung des knappen pro Patient verfügbaren Zeitbudgets mit sich bringt.

Die bislang für solche Zwecke verwendeten Verbindungen erfüllen diese Anforderungen nicht in ausreichendem Maße. In vielen Fällen kommen einfache Schlauchstecksysteme mit einer Schlauchtülle zum Einsatz, die auf ein kegelförmiges Verbindungselement aufgesteckt wird. Bei einer zu geringen und/oder unpräzisen Kraftausübung bei der Herstellung der Verbindung kommt es zu einer zu losen Verbindung oder bei schiefem Aufstecken, so dass die Dichtigkeit unter Umständen nicht mehr in ausreichendem Maße gewährleistet ist und es zum Austreten von Flüssigkeit in die Umgebung oder zum Eindringen von Kontaminationen und Keimen in das Absaugsystem für die Körperflüssigkeit kommen kann. Wird die Verbindung hingegen im Bestreben ebendies zu vermeiden unter hohem Krafteinsatz hergestellt, besteht das Risiko, dass sie nur unter mindestens ebenso hohem Kraftaufwand wieder gelöst werden kann.

Ebenfalls bekannt für die Herstellung solcher Verbindungen sind Luer-Lock-Systeme, bei denen die Dichtwirkung durch eine kegelförmige Konstruktion der Verbindungsteile hergestellt wird und durch das Eindrehen in ein Gewinde die Verbindung gesichert wird. Dabei kommt es aber immer wieder zu Problemen beim Lösen zu fest angezogener Verbindungen; zudem ist die Dichtigkeit nur bei hinreichend präziser Fertigung ausreichend hoch.

Aus der EP 0 633 039 A1 sind ein Verfahren mit den Merkmalen des Oberbegriffs des Anspruchs 1 sowie ein Absaugschlauch und ein Schlauch-Schnellverbinder bekannt. Weiteren Stand der Technik bilden US 2 525 879 A und DE 10 2008 046 098 A.

Die Aufgabe der Erfindung besteht darin, ein Verfahren zur Verbindung eines Absaugschlauchs für Körperflüssigkeiten mit einem Behälter, einen Absaugschlauch zur Durchführung eines solchen Verfahrens und einen Schlauch-Schnellverbinder bereitzustellen, die eine einfachere und zugleich hohen Anforderungen an die Dichtigkeit der Verbindung erfüllenden Herstellung der Verbindung zwischen Absaugschlauch und Behälter ermöglichen.

Diese Aufgabe wird gelöst durch ein Verfahren zur Verbindung eines Absaugschlauchs für Körperflüssigkeiten mit einem Behälter mit den Merkmalen des Patentanspruchs 1, und durch einen Schlauch-Schnellverbinder mit den Merkmalen des Patentanspruchs 5. Vorteilhafte Weiterbildungen der Erfindung sind Gegenstand der abhängigen Patentansprüche.

Das erfindungsgemäße Verfahren zur Verbindung eines Absaugschlauchs für Körperflüssigkeiten mit einem Behälter weist zumindest die folgenden Schritte auf:
- Bereitstellen eines an einem Schlauchadapter angeordneten Schlauchabschnitts, einer Dichtung mit mindestens einer ringförmigen Dichtfläche und eines Behälters, an dem ein Schlauchadapter-Gegenstück befestigt ist.

Bei der Vorgehensweise gemäß diesem erfindungsgemäßen Schritt wird insbesondere dadurch, dass die Verbindungen zwischen Schlauchabschnitt und Schlauchadapter einerseits und Schlauchadapter-Gegenstück und Behälter andererseits herstellerseitig vorkonfiguriert werden das Risiko von durch die Bedienung hervorgerufenen Undichtigkeiten auf die Verbindung zwischen Schlauchadapter und Schlauchadapter-Gegenstück reduziert, wo es durch entsprechende Gestaltung dieser Komponenten besser kontrollierbar ist als z.B. bei der freien Herstellung einer Verbindung zwischen einem Endbereich eines Schlauchabschnitts mit einem kegelförmigen Verbindungselement.

Die Dichtung kann in vielen Fällen einfach ringförmig ausgestaltet sein; es gibt aber auch möglich Konfigurationen, bei denen eine so gebildete ringförmige Dichtfläche (die wie z.B. ein O-Ring eine Dichtung zwischen zwei unterschiedlichen Komponenten herstellen kann, also nicht allein durch die Fläche, mit der die Dichtung mit einer Komponenten wechselwirkt gebildet wird) nur einer der Abschnitte der Dichtung ist, beispielsweise dann, wenn die Dichtung zusätzlich einen rohrförmigen Abschnitt aufweist, mit dem sie auf den Absaugschlauch aufgeschoben und mit diesem verbunden ist.
- Herstellen eines mechanischen Kontakts zwischen einer ersten Anlagefläche, die am Schlauchadapter vorgesehen ist, der Dichtung mit mindestens einer ringförmigen Dichtfläche und einer zweiten Anlagefläche, die am Schlauchadapter-Gegenstück (20) vorgesehen ist in einer ersten Orientierung des Schlauchadapters (10) relativ zum Schlauchadapter-Gegenstück (20), und
- Rotieren des Schlauchadapters aus der ersten Orientierung in eine zweite Orientierung, in der durch eine Wechselwirkung zwischen Abschnitten des Schlauchadapters und Abschnitten des Schlauchadapter-Gegenstücks , so dass die Dichtung mit mindestens einer ringförmigen Dichtfläche zwischen der ersten Anlagefläche und der zweiten Anlagefläche eingequetscht wird.

Durch die letztgenannten Schritte wird einerseits die Verriegelung des Schlauchadapters mit dem Schlauchadapter-Gegenstück hergestellt, die ein unerwünschtes Trennen dieser beiden Komponenten verhindert (da andernfalls keine Quetschkräfte aufgebaut werden könnten). Andererseits geschieht diese auf eine Art und Weise, die die Komponenten mit einer Kraft aufeinander zu beaufschlagt und die Dichtung mit mindestens einer ringförmigen Dichtfläche einquetscht, wodurch einerseits die Dichtwirkung erhöht wird.

Eine bevorzugte Weiterbildung der Erfindung sieht vor, dass beim Herstellen des mechanischen Kontakts ein Abschnitt des Schlauchadapters, in dem zumindest ein Teil einer Durchgangsöffnung, die mit dem Innenraum des Schlauchabschnitts kommuniziert, verläuft, und an dem mindestens ein Verriegelungselement angeordnet ist, in der ersten Orientierung in eine das Schlauchadapter-Gegenstück durchsetzende Öffnung eingeführt wird bis das Verriegelungselement und ein Zugang zur Durchgangsöffnung auf der anderen Seite des Schlauchadapter-Gegenstücks aus der das Schlauchadapter-Gegenstück durchsetzenden Öffnung austreten, wobei während des Einführens des mit dem Teil der mit dem Innenraum des Schlauchabschnitts kommunizierenden Durchgangsöffnung versehenen Abschnitts des Schlauchadapters die Dichtung mit mindestens einer ringförmigen Dichtfläche auf dem mit Teil der mit dem Innenraum des Schlauchabschnitts kommunizierenden Durchgangsöffnung versehenen Abschnitt des Schlauchadapters aufgesteckt und im Raumbereich zwischen einer ersten Anlagefläche, die am Schlauchadapter vorgesehen ist und einer zweiten Anlagefläche, die am Schlauchadapter-Gegenstück vorgesehen ist angeordnet ist.

Durch die Vorgehensweise gemäß dem so weitergebildeten Schritt wird vermieden, dass es zu einer Übergabe der Körperflüssigkeit zwischen Schlauchadapter und Schlauchadaptergegenstück kommen muss, weil sie direkt aus dem Schlauchadapter, genauer gesagt aus der mit dem Innenraum des Schlauchabschnitts kommunizierenden Durchgangsöffnung, in den Behälter übertreten kann. Die Saugrichtung, die die Körperflüssigkeit ja in den Behälter fördern soll und bei üblicher Ausrichtung des Behälterzugangs, die vorzugsweise vertikal ist oder zumindest eine vertikale Komponente aufweist auch die Schwerkraft unterstützen einen direkten Übergang von aus dem durch das Schlauchadapter-Gegenstück hindurchgeführten Abschnitt austretender Körperflüssigkeit in den Behälter und wirken einem Zurücklaufen zu der Stelle, an der ein Austritt in die Umgebung grundsätzlich möglich ist, weil Umgebung, Schlauchadapter und Schlauchadapter-Gegenstück aufeinandertreffen, entgegen. Zudem kann bei entsprechender passgenauer Gestaltung der das Schlauchadapter-Gegenstück durchsetzenden Öffnung und des in sie einzuführenden Abschnitts des Schlauchadapters erreicht werden, dass die Körperflüssigkeit einen langen Kriechweg zurückzulegen hat, ehe sie überhaupt an die Stelle, an der der Austritt in die Umgebung grundsätzlich möglich wäre, gelangt.

Noch weiter ist durch die Positionierung der Dichtung mit mindestens einer ringförmigen Dichtfläche in diesem Schritt sichergestellt, dass sie nach Abschluss des Verfahrens so angeordnet ist, dass auch sie noch dem Übertritt von Körperflüssigkeit in die Umgebung entgegen wirkt.

Besonders bevorzugt ist dabei, wenn beim Rotieren des Schlauchadapters aus der ersten Orientierung in eine zweite Orientierung das Verriegelungselement in eine Position gebracht wird, in der es das Schlauchadapter-Gegenstück in Richtung auf den Schlauchabschnitt hin drückt, so dass die Dichtung mit mindestens einer ringförmigen Dichtfläche zwischen der ersten Anlagefläche und der zweiten Anlagefläche eingequetscht wird.

Anzumerken ist noch, dass natürlich das Rotieren des betreffenden Abschnitts des Schlauchadapters auch gemeinsam mit dem gesamten Schlauchadapter erfolgen kann und im Regelfall auch so erfolgen wird.

Ferner soll nicht unerwähnt bleiben, dass in dieser Konfiguration im Hinblick auf eine Kontamination der Umgebung mit Körperflüssigkeit selbst bei einer nicht perfekt durchgeführten Verbindung ein hoher Grad von Sicherheit erzielt wird, solange die Verriegelungsfunktion erfüllt ist, denn wenn die Dichtung nicht vollständig dicht schließen sollte, wird als Folge der Absaugung Umgebungsluft eingesaugt, so dass ein Fluidstrom entsteht, der einem Kriechen von Körperflüssigkeit in Richtung der Öffnung entgegen wirkt.

Erfindungsgemäß ist vorgesehen, dass zum Bereitstellen des an dem Schlauchadapter angeordneten Schlauchabschnitts ein Endabschnitt des Schlauchabschnitts, dessen innerer Querschnitt dem Querschnitt der Durchgangsöffnung entspricht, in eine sich schlauchseitig an die Durchgangsöffnung anschließende, mit der Durchgangsöffnung konzentrische Ausnehmung, deren Ausdehnung an den äußeren Querschnitt des Schlauchs angepasst ist, eingeschoben wird bis die Stirnseite des Endabschnitts des Schlauchabschnitts auf einer Stufe aufliegt und die Außenwand des Endabschnitts des Schlauchabschnitts an der Wand der Ausnehmung anliegt und mindestens entweder die Stirnseite des Endabschnitts des Schlauchabschnitts oder die Außenwand des Endabschnitts des Schlauchabschnitts fixiert wird.

Diese Vorgehensweise bei der vorzugsweise durch den Hersteller durchgeführten Bereitstellung des mit dem Schlauchadapter versehenen Schlauchabschnitts kann das Risiko des Austretens von abgesaugter Körperflüssigkeit in die Umgebung stark minimieren. Der ringförmige, durch die Wand der Ausnehmung gebildete Mantel schützt das in die Ausnehmung eingeführte Ende des Schlauchabschnitts vor mechanischer Einwirkung von außen, während er gleichzeitig auch ein unmittelbares Entweichen von abgesaugter Körperflüssigkeit in die Umgebung verhindert. Wird der Schlauchabschnitt fixiert, kann dies insbesondere durch stoffschlüssige Verbindung mit einem Kleber oder -vorzugsweise aushärtendem- Dichtmaterial geschehen kann, ist die Dichtigkeit dieser Verbindung quasi garantiert. Erfindungsgemäß wird beim Rotieren des mit dem Innenraum des Schlauchs kommunizierenden Durchgangsöffnung versehenen Abschnitts des Schlauchadapters aus der ersten Orientierung in eine zweite Orientierung das Verriegelungselement zumindest während Teilen der Rotation entlang einer dritten Anlagefläche geführt, deren Abstand zur zweiten Anlagefläche von der Position des Verriegelungselements ausgehend von der ersten Orientierung hin zur Position des Verriegelungselements in der zweiten Orientierung wächst.

Während der Teile der Rotation, während denen dieses Merkmal erfüllt ist, wird als Konsequenz daraus der Abstand zwischen erster Anlagefläche, also der am Schlauchadapter befindlichen, dem Schlauchadapter-Gegenstück zugewandten Anlagefläche und zweiter Anlagefläche, also der am Schlauchadapter-Gegenstück befindlichen, dem Schlauchabschnitt zugewandten Anlagefläche verringert und die Position der Dichtung mit mindestens einer ringförmigen Dichtfläche definiert, bis sie zwischen der ersten und zweiten Anlagefläche eingequetscht ist.

Besonders bevorzugt ist auch eine Weiterbildung des Verfahrens, bei der beim Rotieren des mit dem Innenraum des Schlauchs kommunizierenden Durchgangsöffnung versehenen Abschnitts des Schlauchadapters aus der ersten Orientierung in eine zweite Orientierung das Verriegelungselement vor dem Erreichen der zweiten Orientierung eine Position durchläuft, in der die Dichtung mit mindestens einer ringförmigen Dichtfläche stärker zusammengequetscht ist als in der zweiten Orientierung. Besonders bevorzugt wird diese Position unmittelbar vor dem Erreichen der zweiten Orientierung durchlaufen. Diese Maßnahme bringt den Effekt mit sich, dass der Schlauchadapter in der zweiten Position fixiert ist.

Der die ringförmige Dichtfläche aufweisende Abschnitt der Dichtung, die besonders vozugsweise insgesamt, vorzugsweise zumindest in diesem Abschnitt elastisch sein sollte, um diesen Effekt so gut wie möglich auszunutzen, erfüllt dann neben der Dichtungsfunktion auch eine Funktion als Feder, vergleichbar der Funktion der Tellerfedern, die bei bekannten Vierteldreh-Schnellverschlüssen zur Verbindung von Bauteilen miteinander zum Einsatz kommen.

Ein nicht zur beanspruchten Erfindung gehörender Absaugschlauch zur Durchführung des Verfahrens ist mit einem Schlauchabschnitt und mit einem Schlauchadapter, der an einem Endabschnitt des Schlauchabschnitts angeordnet ist, ausgestattet. Dabei weist der Schlauchadapter mindestens ein Verriegelungselement oder mindestens eine Nut auf, die derart ausgelegt ist, dass beim Rotieren des Schlauchadapters aus einer ersten Orientierung in eine zweite Orientierung durch eine Wechselwirkung zwischen dem Verriegelungselement oder der Nut des Schlauchadapters einerseits und einer Nut oder eines Verriegelungselements des Schlauchadapter-Gegenstücks andererseits eine Dichtung mit mindestens einer ringförmigen Dichtfläche zwischen einer ersten, am Schlauchadapter vorhandenen Anlagefläche und einer am Schlauchadapter-Gegenstück angeordneten zweiten Anlagefläche eingequetscht wird.

Eine nicht zur beanspruchten Erfindung gehörende Ausführungsform des Absaugschlauchs zur Durchführung eines solchen Verfahrens hat einen Schlauchabschnitt und einen Schlauchadapter, der an einem Endabschnitt des Schlauchabschnitts angeordnet ist, wobei der Schlauchadapter einen mit einer mit dem Innenraum des Schlauchabschnitts kommunizierenden Durchgangsöffnung versehenen Abschnitt zum Hindurchführen durch eine Öffnung eines Schlauchadapter-Gegenstücks aufweist, an dem mindestens ein Verriegelungselement angeordnet ist.

Durch diesen Abschnitt wird sichergestellt, dass die Durchgangsöffnung des Schlauchadapters innerhalb eines Behälters, an dem das Schlauchadapter-Gegenstück angebracht ist, angeordnet ist, was ein Austreten von Körperflüssigkeit in die Umgebung signifikant erschwert und die Anforderungen an die Herstellung der Verbindung zwischen Schlauchadapter und Schlauchadaptergegenstück vereinfacht.

In einer nicht zur beanspruchten Erfindung gehörenden Weiterbildung eines solchen Absaugschlauchs ist vorgesehen, dass der innere Querschnitt des Endabschnitts des Schlauchabschnitts dem Querschnitt der Durchgangsöffnung entspricht und dass der Endabschnitt des Schlauchabschnitts in einer sich schlauchseitig an die Durchgangsöffnung anschließenden, mit der Durchgangsöffnung konzentrischen Ausnehmung, deren Ausdehnung an den äußeren Querschnitt des Endabschnitts des Schlauchabschnitts angepasst ist angeordnet ist, so dass die Stirnseite des Endabschnitts des Schlauchabschnitts auf einer Stufe aufliegt und die Außenwand des Endabschnitts des Schlauchabschnitts an der Wand der Ausnehmung anliegt und mindestens entweder die Stirnseite des Endabschnitts des Schlauchabschnitts oder die Außenwand des Endabschnitts des Schlauchabschnitts fixiert ist.

Dabei schützt die Wandung der Ausnehmung den Endabschnitt des Schlauchabschnitt vor mechanischer Einwirkung und stellt durch ihre Ausgestaltung einen flächigen Kontakt zwischen dem Ende des Schlauchabschnitts und dem Schlauchadapter sicher, der das Risiko von Undichtigkeiten signifikant reduziert.

Wird der Schlauchabschnitt fixiert, was bevorzugt stoffschlüssig erfolgt, insbesondere mit einem Kleber oder -vorzugsweise aushärtendem- Dichtungsmaterial, ist die Dichtigkeit dieser Verbindung quasi garantiert.

Gemäß einer anderen nicht zur beanspruchten Erfindung gehörenden Weiterbildung des Absaugschlauchs sind an dem Schlauchadapter Hilfselemente zur Unterstützung der Krafteinleitung beim Herbeiführen einer Rotationsbewegung des Schlauchadapters um eine durch die Durchgangsöffnung definierte Achse herum vorhanden sind, die insbesondere dann wenn ein ringförmiger Mantel vorhanden ist als Vorsprünge, Ausstülpungen oder Einbuchtungen in diesem ringförmigen Mantel realisiert sein können.

Auf diese Weise kann auch älteren Personen, die keine hohen Griffkräfte mehr aufbringen können, das Herstellen einer dichten und zuverlässigen Verbindung spürbar erleichtert werden.

Der erfindungsgemäße Schlauch-Schnellverbinder weist einen Schlauchadapter, eine Dichtung mit mindestens einer ringförmigen Dichtfläche und ein Schlauchadapter-Gegenstück, das von einer Öffnung zur Aufnahme eines Abschnitts des Schlauchadapters in mindestens einer Orientierung des Schlauchadapters durchsetzt wird, auf.

Dabei hat der Schlauchadapter auf seiner bestimmungsgemäß dem Schlauch zugewandten Seite einen Schlauchanschluss zur Verbindung mit einem Schlauchabschnitt und auf der bestimmungsgemäß dem Schlauchadapter-Gegenstück zugewandten Seite eine erste Anlagefläche für die Dichtung mit mindestens einer ringförmigen Dichtfläche.

Ferner weist das Schlauchadapter-Gegenstück eine zweite Anlagefläche für die Dichtung mit mindestens einer ringförmigen Dichtfläche auf seiner bestimmungsgemäß dem Schlauchadapter zugewandten Seite auf, und Schlauchadapter und Schlauchadapter-Gegenstück weisen jeweils Mittel auf, die derart miteinander wechselwirken, dass durch Rotation des Schlauchadapters relativ zum Schlauchadapter-Gegenstück von einer ersten Orientierung zu einer zweiten Orientierung, die Dichtung mit mindestens einer ringförmigen Dichtfläche zwischen der ersten Anlagefläche und der zweiten Anlagefläche eingequetscht wird.

Gemäß einer bevorzugten Weiterbildung des Schlauch-Schnellverbinders ist vorgesehen, dass das Schlauchadapter-Gegenstück von einer Öffnung zur Aufnahme eines Abschnitts des Schlauchadapters in mindestens einer Orientierung des Schlauchadapters durchsetzt wird, dass der Schlauchadapter eine Durchgangsöffnung, die mit dem Innenraum des Schlauchabschnitts kommuniziert, und einen Abschnitt zum Hindurchführen durch die Öffnung im Schlauchadapter-Gegenstück zur Aufnahme eines Abschnitts des Schlauchadapters in mindestens einer Orientierung des Schlauchadapters aufweist, an dem mindestens ein Verriegelungselement angeordnet ist und in dem zumindest ein Teil der Durchgangsöffnung, die mit dem Innenraum des Schlauchabschnitts kommuniziert, verläuft. Ferner ist vorgesehen, dass die Öffnung zur Aufnahme eines Abschnitts des Schlauchadapters in mindestens einer Orientierung des Schlauchadapters und der Abschnitt des Schlauchadapters zum Hindurchführen durch die Öffnung zur Aufnahme des Abschnitts des Schlauchadapters derart aufeinander abgestimmt sind, dass das Verriegelungselement durch Rotation des Schlauchadapters aus einer ersten Orientierung, in der es durch die Öffnung zur Aufnahme eines Abschnitts des Schlauchadapters hindurchführbar ist, in eine zweite Orientierung, in der das Verriegelungselement das Schlauchadapter-Gegenstück in Richtung auf den Schlauchabschnitt hin drückt, überführbar ist.

Durch diese Ausgestaltung des Schlauch-Schnellverbinders wird einerseits vermieden, dass es zu einer Übergabe der Körperflüssigkeit zwischen Schlauchadapter und Schlauchadaptergegenstück kommen muss, weil sie direkt aus dem Schlauchadapter, genauer gesagt aus der mit dem Innenraum des Schlauchabschnitts kommunizierenden Durchgangsöffnung, in den Behälter übertreten kann. Die Saugrichtung, die die Körperflüssigkeit ja in den Behälter fördern soll und bei üblicher Ausrichtung des Behälterzugangs, die vorzugsweise vertikal ist oder zumindest eine vertikale Komponente aufweist auch die Schwerkraft unterstützen einen direkten Übergang von aus dem durch das Schlauchadapter-Gegenstück hindurchgeführten Abschnitt austretender Körperflüssigkeit in den Behälter und wirken einem Zurücklaufen zu der Stelle, an der ein Austritt in die Umgebung grundsätzlich möglich ist, weil Umgebung, Schlauchadapter und Schlauchadapter-Gegenstück aufeinandertreffen, entgegen.

Zudem kann bei entsprechender passgenauer Gestaltung der das Schlauchadapter-Gegenstück durchsetzenden Öffnung und des in sie einzuführenden Abschnitts des Schlauchadapters erreicht werden, dass die Körperflüssigkeit einen langen Kriechweg zurückzulegen hat, ehe sie überhaupt an die Stelle, an der der Austritt in die Umgebung grundsätzlich möglich wäre, gelangt.

Noch weiter ist durch die Positionierung der Dichtung mit mindestens einer ringförmigen Dichtfläche bei diesem Schlauch-Schnellverbinder im verbundenen Zustand von dessen Bestandteilen sichergestellt, dass auch sie noch dem Übertritt von Körperflüssigkeit in die Umgebung entgegen wirkt.

Zudem wird bei diesem Schlauch-Schnellverbinder eine Verriegelung des Schlauchadapters mit dem Schlauchadapter-Gegenstück hergestellt, die ein unerwünschtes Trennen dieser beiden Komponenten verhindert. Zugleich geschieht diese Verriegelung auf eine Art und Weise, die die Komponenten mit einer Kraft aufeinander zu beaufschlagt und die Dichtung mit mindestens einer ringförmigen Dichtfläche einquetscht, wodurch die Dichtwirkung weiter erhöht wird.

Bei einer bevorzugten Weiterbildung des Schlauch-Schnellverbinders ist vorgesehen, dass der innere Querschnitt des Endabschnitts des Schlauchabschnitts dem Querschnitt der Durchgangsöffnung entspricht und dass der Endabschnitt des Schlauchabschnitts in einer sich schlauchseitig an die Durchgangsöffnung anschließenden, mit der Durchgangsöffnung konzentrischen Ausnehmung, deren Ausdehnung an den äußeren Querschnitt des Endabschnitts des Schlauchabschnitts angepasst ist angeordnet ist, so dass die Stirnseite des Endabschnitts des Schlauchabschnitts auf einer Stufe aufliegt und die Außenwand des Endabschnitts des Schlauchabschnitts an der Wand der Ausnehmung anliegt und mindestens entweder die Stirnseite des Endabschnitts des Schlauchabschnitts oder die Außenwand des Endabschnitts des Schlauchabschnitts fixiert ist. Wird der Schlauchabschnitt stoffschlüssig fixiert, insbesondere durch das Füllen des Ringspalts mit einem Kleber oder -vorzugsweise aushärtendem- Dichtungsmaterial, dann die Dichtigkeit dieser Verbindung quasi garantiert.

Ebenfalls ist es bevorzugt, wenn an dem Schlauchadapter Hilfselemente zur Unterstützung der Krafteinleitung beim Herbeiführen einer Rotationsbewegung des Schlauchadapters um eine durch die Durchgangsöffnung definierte Achse herum vorhanden sind. Die Hilfselemente können insbesondere dann wenn ein ringförmiger Mantel vorhanden ist als Vorsprünge, Ausstülpungen oder Einbuchtungen in diesem ringförmigen Mantel realisiert sein. Auf diese Weise kann auch älteren Personen, die keine hohen Griffkräfte mehr aufbringen können, das Herstellen einer dichten und zuverlässigen Verbindung spürbar erleichtert werden.

In einer bevorzugten Ausführungsform des Schlauch-Schnellverbinders wird das Zusammenquetschen der Dichtung mit mindestens einer ringförmigen Dichtfläche dadurch erreicht, dass an dem Schlauchadapter-Gegenstück eine dritte Anlagefläche vorhanden ist, deren Abstand zur zweiten Anlagefläche von der Position des Verriegelungselements ausgehend von der ersten Orientierung hin zur Position des Verriegelungselements in der zweiten Orientierung wächst und dass beim Rotieren des mit dem Innenraum des Schlauchs kommunizierenden Durchgangsöffnung versehenen Abschnitts des Schlauchadapters aus der ersten Orientierung in die zweite Orientierung das Verriegelungselement zumindest abschnittsweise -also nicht zwingend während der gesamten Bewegung- entlang dieser dritten Anlagefläche geführt wird.

Erfindungsgemäß ist es, wenn in beim Rotieren des mit der Durchgangsöffnung versehenen Abschnitts des Schlauchadapters aus der ersten Orientierung in eine zweite Orientierung das Verriegelungselement vor dem Erreichen der zweiten Orientierung auf einer solchen Anlagefläche eine Position durchläuft, in der die Dichtung mit mindestens einer ringförmigen Dichtfläche stärker zusammengequetscht ist als in der zweiten Orientierung. Besonders bevorzugt wird diese Position unmittelbar vor dem Erreichen der zweiten Orientierung durchlaufen. Diese Maßnahme bringt den Effekt mit sich, dass der Schlauchadapter in der zweiten Position fixiert ist. Der die ringförmige Dichtfläche aufweisende Abschnitt der Dichtung, die besonders vorzugsweise insgesamt, vorzugsweise zumindest in diesem Abschnitt elastisch sein sollte, um diesen Effekt so gut wie möglich auszunutzen, erfüllt dann neben der Dichtungsfunktion auch eine Funktion als Feder, vergleichbar der Funktion der Tellerfedern, die bei bekannten Vierteldreh-Schnellverschlüssen zur Verbindung von Bauteilen miteinander zum Einsatz kommen.

Die Erfindung wird nachfolgend anhand von Figuren, die ein Ausführungsbeispiel zeigen, näher erläutert. Es zeigt:
- Fig. 1:: Eine Explosionszeichnung eines Schlauch-Schnellverbinders,
- Fig. 2:: den Schlauch-Schnellverbinder aus Figur 1 in einem offenen Zustand mit in das Schlauchadapter-Gegenstück eingeführtem Abschnitt des Schlauchadapters,
- Fig. 3:: den Schlauch-Schnellverbinder aus Figur 1 in einem Zwischenzustand beim Schließen des Schlauch-Schnellverbinders,
- Fig. 4:: einen Querschnitt durch Figur 3 längs der Linie B-B,
- Fig. 5:: eine Explosionszeichnung einer ersten Variante des Schlauch-Schnellverbinders aus Figur 1,
- Fig. 6:: eine Explosionszeichnung einer zweiten Variante des Schlauch-Schnellverbinders aus Figur 1,
- Fig. 7:: eine Explosionszeichnung eines anderen Schlauch-Schnellverbinders,
- Fig. 8:: eine Seitenansicht des Schlauch-Schnellverbinders aus Figur 7, und
- Fig. 9:: einen Querschnitt durch Figur 8 längs der Linie B-B.

Soweit in Figuren um dasselbe Ausführungsbeispiel dargestellt ist, werden dieselben Bezugszeichen verwendet, sofern nichts anderes erwähnt ist. Allerdings sind zur Verbesserung der Übersichtlichkeit nicht alle Bezugszeichen in allen Figuren eingetragen.

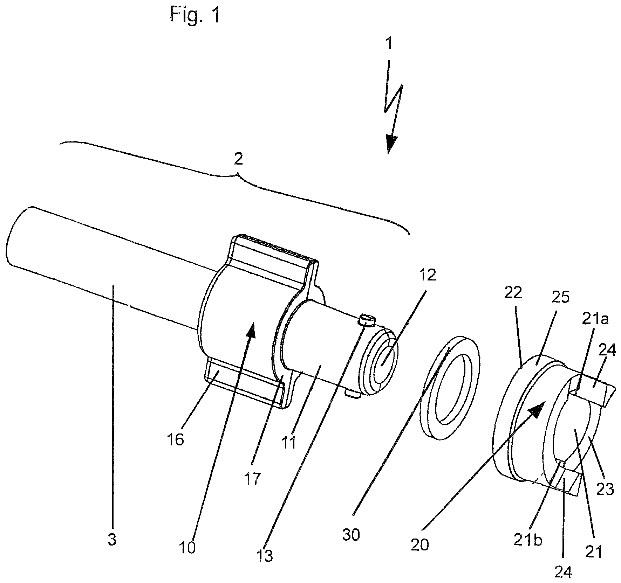

Die Figuren 1 bis 4 zeigen unterschiedliche Ansichten eines Schlauch-Schnellverbinder 1, bestehend aus einem Schlauchadapter 10, einem Schlauchadapter-Gegenstück 20 und einer Dichtung mit mindestens einer ringförmigen Dichtfläche 30, wobei diese als Dichtring ausgestaltet ist. Dabei bilden der Schlauchadapter 10 und ein an ihm angeordneter Schlauchabschnitt 3 zusammen einen Absaugschlauch 2, der im praktischen Anwendungsfall mit dem Schlauchadapter-Gegenstück 20 verbunden wird, um unter Unterdruck abgesaugte Körperflüssigkeit in einen nicht dargestellten Behälter, an dem das Schlauchadapter-Gegenstück 20 vorzugsweise in der Praxis angeordnet ist, zu überführen.

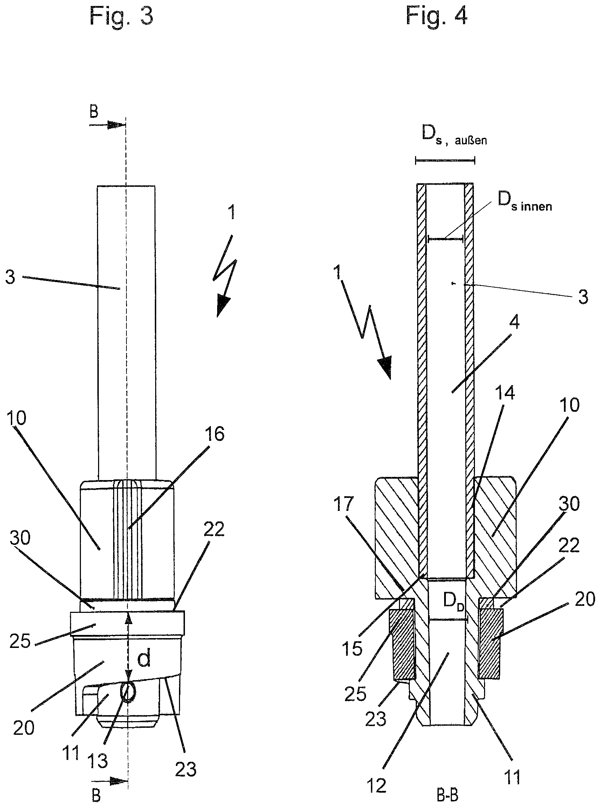

Der Aufbau des Schlauchadapters 10 lässt sich am besten in Zusammenschau der Explosionsdarstellung gemäß Figur 1 mit der Schnittdarstellung gemäß Figur 4 erkennen. Der Schlauchadapter 10 weist einen Abschnitt 11 auf, der von einer Durchgangsöffnung 12 durchsetzt wird und in seinem Endbereich an der bestimmungsgemäß vom Schlauchabschnitt 3 abgewandten Seite zwei Verriegelungselemente 13, die hier als sich radial nach außen erstreckende Zapfen ausgeführt sind, auf. Am dem Ende des Abschnitts 11 das dem Schlauchabschnitt 3 zugewandt ist, wird der Abschnitt 11 in radialer Richtung vom angrenzenden Bereich des Schlauchadapters 10 überragt, so dass eine erste Anlagefläche 17 gebildet wird.

Schlauchseitig geht die Durchgangsöffnung 12, deren Durchmesser D_{D} an den Innendurchmesser D_{S,innen} des Innenraums 4 des Schlauchabschnitts 3 angepasst ist, in eine Ausnehmung 14 über, deren Durchmesser D_{A} an den Außendurchmesser D_{S,außen} des Schlauchabschnitts 3 angepasst ist. Dementsprechend ist eine Stufe 15 vorhanden, auf der die Stirnseite der Wände des Endabschnitts des Schlauchabschnitts 3 aufliegt, während die Außenseite dieser Wände an der Innenseite der Wände der Ausnehmung 14 anliegen.

Grundsätzlich kann bereits hierdurch eine hinreichende Abdichtung gegenüber Flüssigkeitsaustritt und eine ausreichende Fixierung des Schlauchs erreicht werden; weiter verbessert werden diese Eigenschaften aber durch das Schaffen einer stoffschlüssigen Verbindung, z.B. durch Verkleben zwischen der Stufe 15 bzw. den Wänden der Ausnehmung 14 und der Stirnseite bzw. Außenwand des Schlauchabschnitts 3.

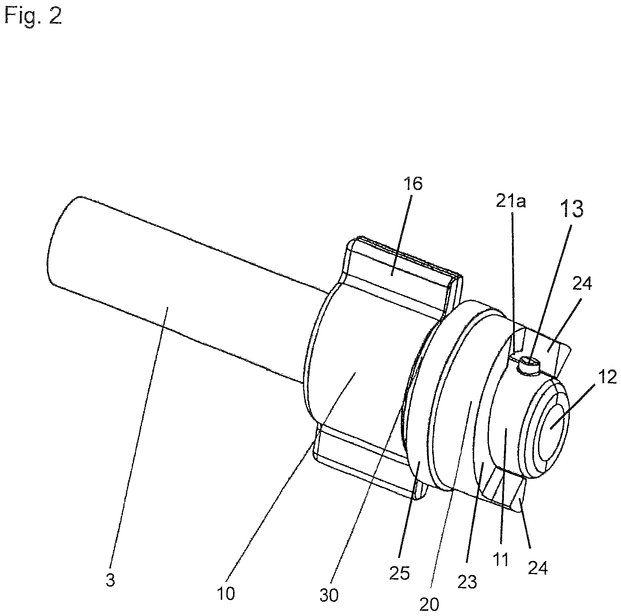

Um den Schlauchadapter 10 nach dem Einführen des Abschnitts 11 in die das Schlauchadapter-Gegenstück 20 durchsetzende Öffnung 21 leichter durch Rotation aus der in Figur 2 gezeigten ersten Orientierung, in der der Abschnitt 11 in die Öffnung 21 einführbar ist, in die zweite Orientierung, in der er die Dichtung mit mindestens einer ringförmigen Dichtfläche 30 zusammenquetscht, überführen zu können, weist er Hilfselemente 16, die hier als zwei einander gegenüberliegende, sich in radialer Richtung nach außen erstreckende Vorsprünge des Schlauchadapters 10 realisiert sind, auf.

Die das Schlauchadapter-Gegenstück 20 durchsetzende Öffnung 21 hat einen im Wesentlichen kreisförmigen Querschnitt, der jedoch zwei einander gegenüberliegende, sich in radialer Richtung über den Radius des dem kreisförmigen Querschnitt entsprechenden Kreises hinaus erstreckende Ausnehmungen 21a,21b aufweist, um das Einführen des Abschnitts 11 mitsamt der an ihm angeordneten Verriegelungselemente 13 in der ersten Orientierung durch die Öffnung 21 hindurchführen zu können.

Die dem Schlauchabschnitt 3 zugewandte Stirnfläche des Schlauchadapter-Gegenstücks 20 bildet eine zweite Anlagefläche 22, die in den Figuren 3 und 4 besonders gut zu erkennen ist. Die der zweiten Anlagefläche 22 gegenüber liegenden Stirnfläche des Schlauchadapter-Gegenstücks 20 bildet eine dritte Anlagefläche 23, entlang der die Verriegelungselemente 13 nachdem sie ein Stück weit aus der ersten Orientierung herausrotiert wurden geführt werden, und zwar maximal bis sie ihre durch Vorsprünge 24, die jeweils einen Anschlag für ein Verriegelungselement 13 bilden, definierte Endposition erreichen.

Zu betonen ist dabei, dass die zweite Orientierung, in der das Verriegelungselement 13 sich in einer Position befindet, in der es das Schlauchadapter-Gegenstück 20 in Richtung auf den Schlauchabschnitt 3 hin drückt, so dass die Dichtung mit mindestens einer ringförmigen Dichtfläche 30 zwischen der ersten Anlagefläche 17 und der zweiten Anlagefläche 22 eingequetscht wird, je nach Ausgestaltung der dritten Anlagefläche 23 bereits vor dem Erreichen des durch die Vorsprünge 24 gebildeten Anschlags liegen kann.

Die Anlageflächen 23 sind dabei, wie in Figur 3 besonders gut sichtbar ist, derart ausgestaltet, dass auf dem Weg der Bewegung der Verriegelungselemente 13 der in Figur 3 bei der dort aktuellen Position der Verriegelungselemente 13 exemplarisch eingetragene Abstand d zwischen der zweiten Anlagefläche 22 zur dritten Anlagefläche zunimmt, so dass das Schlauchadapter-Gegenstück 20 durch die Verriegelungselemente 13 in Richtung auf den Schlauchabschnitt 3 gedrückt wird und dadurch die Dichtung mit mindestens einer ringförmigen Dichtfläche 30 zunehmend eingequetscht wird.

Der Außendurchmesser des Schlauchadapter-Gegenstücks 20 kann dabei an den Innendurchmesser einer Behälteröffnung eines nicht dargestellten Behälters angepasst sein und auf der dem Schlauchabschnitt 3 zugewandten Seite einen radial vorspringenden Abschnitt 25 aufweisen, der diesen Innendurchmesser überragt.

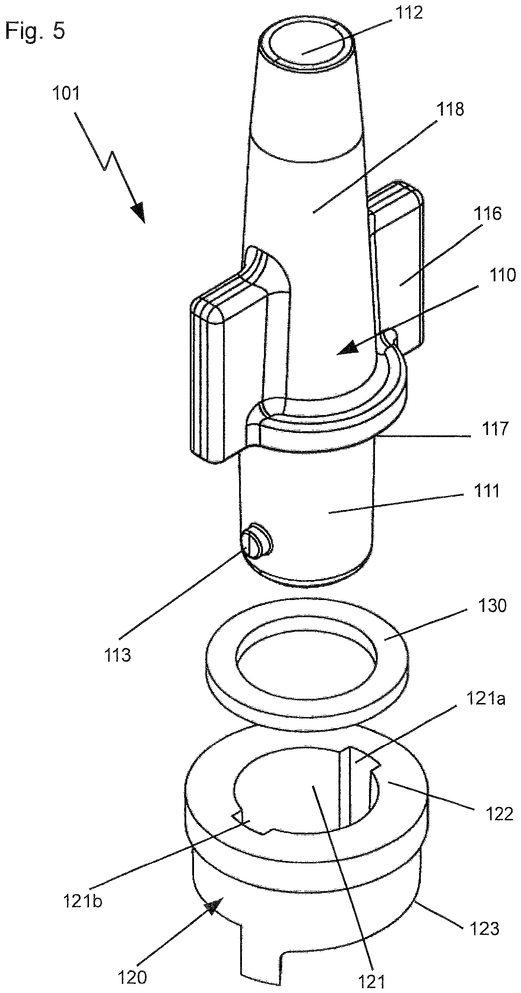

Die Figur 5 zeigt eine Explosionsdarstellung eines Schlauch-Schnellverbinders 101, der eine Variante des Schlauch-Schnellverbinders 1 ist und ebenfalls aus einem Schlauchadapter 110, einem Schlauchadapter-Gegenstück 120 und einer Dichtung mit mindestens einer ringförmigen Dichtfläche 130, die als Dichtring ausgestaltet ist, besteht.

Der Aufbau des Schlauch-Schnellverbinders 101 weicht vom Aufbau des Schlauch-Schnellverbinders 1 hinsichtlich der Art und Weise ab, die die Verbindung zum nicht dargestellten Schlauchabschnitt hergestellt wird. Dementsprechend kann weitgehend die Beschreibung des Schnellverbinders 1 ergänzend zur nachfolgenden Beschreibung beigezogen werden, wobei die Bezugszeichen in Figur 5 sich durch Addition der Zahl Hundert zu Bezugszeichen der Figuren 1 bis 4 ergeben.

Der Schlauchadapter 110 weist einen Abschnitt 111 auf, der von einer Durchgangsöffnung 112 durchsetzt wird und in seinem Endbereich an der bestimmungsgemäß vom Schlauchabschnitt abgewandten Seite zwei Verriegelungselemente 113, die hier als sich radial nach außen erstreckende Zapfen ausgeführt sind, auf. Am dem Ende des Abschnitts 111 das dem Schlauchabschnitt zugewandt ist, wird der Abschnitt 111 in radialer Richtung vom angrenzenden Bereich des Schlauchadapters 110 überragt, so dass eine erste Anlagefläche 117 gebildet wird.

Anders als in der Ausführungsform gemäß Figuren 1 bis 4 ist hier aber nur ein Teil der Durchgangsöffnung 112 im Abschnitt 111 angeordnet; sie setzt sich innerhalb des Schlauchadapters 110 in Richtung auf den nicht dargestellten Schlauchabschnitt fort, wo ihre Wandung durch einem Aufsteckkonus 118 gebildet wird.

Im unteren, dem Abschnitt 111 zugewandten Bereich des Aufsteckkonus sind Hilfselemente 116, die dieselbe Funktion wie oben beschrieben haben, angeformt. Auf den Bereich des Aufsteckkonus 118 oberhalb der Hilfselemente 116 wird der nicht dargestellte Schlauchabschnitt aufgesteckt. Vorzugsweise geschieht dies herstellerseitig einmal bei der Herstellung des Anschlussschlauchs, wobei eine stoffschlüssige Verbindung hergestellt wird. Auf diese Weise wird ein sehr einfacher und materialsparender Aufbau des Schlauchadapters 110 ermöglicht, während gleichzeitig die üblichen Probleme bei Verwendung von Aufsteckkonen, bei denen der Benutzer den Schlauch zum Behälterwechsel selbst aufstecken muss, sicher vermieden werden.

Wie bei dem Schlauch-Schnellverbinder 1 hat auch beim Schlauch-Schnellverbinder 101 die das Schlauchadapter-Gegenstück 120 durchsetzende Öffnung 121 hat einen im Wesentlichen kreisförmigen Querschnitt, der zwei einander gegenüberliegende, sich in radialer Richtung über den Radius des dem kreisförmigen Querschnitt entsprechenden Kreises hinaus erstreckende Ausnehmungen 121a,121b aufweist, um das Einführen des Abschnitts 111 mitsamt der an ihm angeordneten Verriegelungselemente 113 in der ersten Orientierung durch die Öffnung 121 zu ermöglichen.

Die dem nicht dargestellten Schlauchabschnitt zugewandte Stirnfläche des Schlauchadapter-Gegenstücks 120 bildet eine zweite Anlagefläche 122. Die der zweiten Anlagefläche 122 gegenüber liegenden Stirnfläche des Schlauchadapter-Gegenstücks 120 bildet eine dritte Anlagefläche 123, entlang der die Verriegelungselemente 113 nachdem sie ein Stück weit aus der ersten Orientierung herausrotiert wurden geführt werden. Auch hier sind die Anlageflächen 123 so ausgestaltet, dass auf dem Weg der Bewegung der Verriegelungselemente der Abstand zwischen der zweiten Anlagefläche 122 und der dritten Anlagefläche 123 zunimmt, so dass das Schlauchadapter-Gegenstück 120 durch die Verriegelungselemente 113 in Richtung auf den Schlauchabschnitt gedrückt wird und dadurch die als kreisringförmige Dichtscheibe ausgeführte Dichtung mit mindestens einer ringförmigen Dichtfläche 130 zunehmend eingequetscht wird.

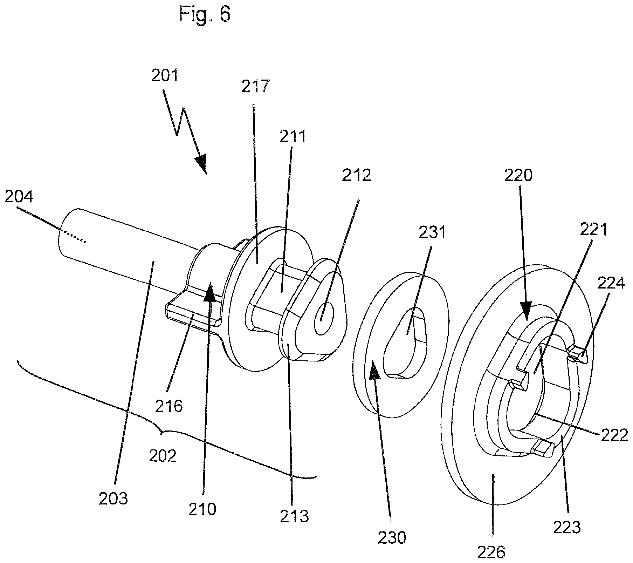

Die Figur 6 zeigt eine Explosionsdarstellung eines Schlauch-Schnellverbinders 201, der eine andere Variante des Schlauch-Schnellverbinders 1 ist und ebenfalls aus einem Schlauchadapter 210, einem Schlauchadapter-Gegenstück 220 und einer Dichtung mit mindestens einer ringförmigen Dichtfläche 230, die hier als Dichtring mit modifizierter Innenkontur ausgestaltet ist, besteht sowie einen Endbereich des Absaugschlauchs 202, mit Schlauchabschnitt 203 und Schlauchadapter 210.

Der Aufbau des Schlauch-Schnellverbinders 201 weicht vom Aufbau des Schlauch-Schnellverbinders 1 hinsichtlich der Ausgestaltung des Abschnitts 211 ab, der durch die Öffnung 221 des Schlauchadapter-Gegenstücks 220 hindurchgeführt wird. Dementsprechend kann weitgehend die Beschreibung des Schnellverbinders 1 ergänzend zur nachfolgenden Beschreibung beigezogen werden, wobei die Bezugszeichen in Figur 5 sich durch Addition der Zahl Zweihundert zu Bezugszeichen der Figuren 1 bis 4 ergeben.

Der Schlauchadapter 210 weist einen Abschnitt 211 auf, dessen Außenkontur die Form eines Dreiecks mit abgerundeten Ecken aufweist und der von einer Durchgangsöffnung 212 durchsetzt wird. Im Endbereich des Abschnitts 211 ist ein Verriegelungselement 213 ausgebildet, das den Abschnitt 211 in radialer Richtung überragt und dessen Außenkontur ein zur Außenkontur des Abschnitts 211 ähnliches Dreieck mit abgerundeten Ecken ist, das aber eine größere Seitenlänge aufweist. Die Innenkontur 231 der Dichtung mit mindestens einer ringförmigen Dichtfläche 230 ist dabei so geformt, dass das Verriegelungselement 213 in einer ersten Orientierung durch das durch die Innenkontur definierte lichte Volumen hindurchgeführt werden kann.

An dem Ende des Abschnitts 211 das dem Schlauchabschnitt 203 zugewandt ist, wird der Abschnitt 211 in radialer Richtung vom angrenzenden Bereich des Schlauchadapters 210 überragt, so dass eine erste Anlagefläche 217 gebildet wird. Die schlauchseitige Gestaltung des Schlauchadapters 210 einschließlich der Hilfselemente 216 und der Art, wie die Verbindung mit dem Schlauchabschnitt 203 hergestellt wird, ist identisch zu der des Schlauchadapters 10, weshalb hier auf die oben stehenden, entsprechenden Passagen der Beschreibung des Schlauchadapters 10 verwiesen werden kann.

Die das Schlauchadapter-Gegenstück 220 durchsetzende Öffnung 221 hat einen Querschnitt, der an die Außenkontur des Verriegelungselements 213 angepasst ist, um das Einführen des Abschnitts 211 mitsamt der an ihm angeordneten Verriegelungselemente 213 in der ersten Orientierung durch die Öffnung 221 hindurchführen zu können.

Die dem Schlauchabschnitt 203 zugewandte Stirnfläche des Schlauchadapter-Gegenstücks 220 bildet eine hier nicht sichtbare zweite Anlagefläche. Sie geht in diesem Ausführungsbeispiel, in dem das Schlauchadapter-Gegenstück 220 in einen Abschnitt 226 der Behälterwand integriert ist und plan mit dieser abschließt, über.

Die der zweiten Anlagefläche 222 gegenüber liegenden Stirnfläche des Schlauchadapter-Gegenstücks 220, die relativ zum Abschnitt 226 der Behälterwand nach innen gezogen ist, bildet eine dritte Anlagefläche 223, entlang der die Verriegelungselemente 213 nachdem sie ein Stück weit aus der ersten Orientierung herausrotiert wurden geführt werden, und zwar maximal bis sie ihre durch Vorsprünge 224, die jeweils einen Anschlag für ein Verriegelungselement 213 bilden, definierte Endposition erreichen.

Zu betonen ist dabei, dass bereits relativ bald nach dem Verlassen der ersten Orientierung die abgerundeten Ecken des Verriegelungselements 213, einen Formschluss mit dem Schlauchadapter-Gegenstück 220 zu bilden beginnen. Insbesondere kann dabei durch einen entsprechend orientierte Fase Druck aufgebaut werden, so dass sich das Verriegelungselement 213 dann in einer Position befindet, in der es das Schlauchadapter-Gegenstück 220 in Richtung auf den Schlauchabschnitt 203 hin drückt, so dass die Dichtung mit mindestens einer ringförmigen Dichtfläche 230 zwischen der ersten Anlagefläche 217 und der zweiten Anlagefläche 222 eingequetscht wird, und zwar bereits ehe ein Anschlag 224 erreicht ist.

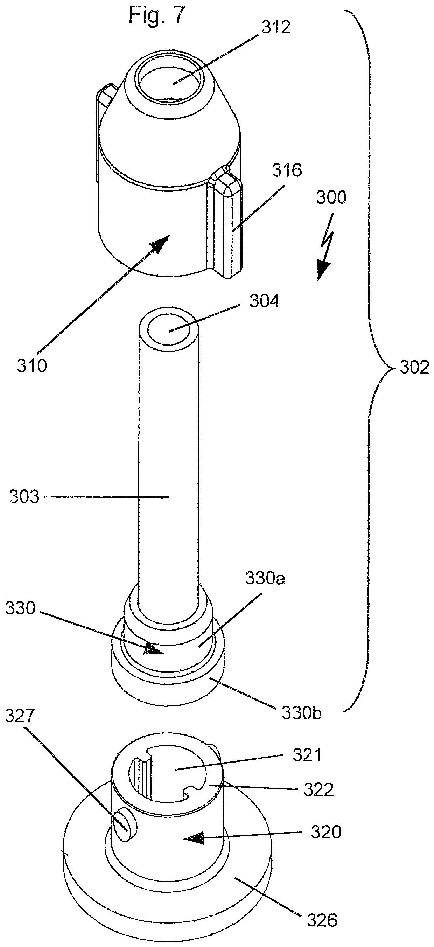

Die Figuren 7 bis 9 zeigen ein Ausführungsbeispiel eines im Vergleich zu den bisher diskutierten Schlauch-Schnellverbindern 1,101,201 deutlich anders ausgestalteten Schlauch-Schnellverbinder 300 bzw. Absaugschlauch 302. In diesem Fall weist der Schlauchabschnitt 303 an seinem einen Ende eine Dichtung 330 auf, die ihrerseits einen hülsenförmigen Halteabschnitt 330a und eine ringförmige Dichtfläche 330b aufweist.

Die ringförmige Dichtfläche 330b wird mit ihrer dem Schlauchadapter-Gegenstück 320, das in diesem Beispiel außen an einem Abschnitt 326 des Behälters angeformt ist und zapfenförmige Verriegelungselemente 327 aufweist, zugewandten Seite auf der durch die freie Stirnseite des Schlauchadapter-Gegenstücks 320 gebildete zweite Anlagefläche 322, die dessen Öffnung 321 im Wesentlichen ringförmig umgibt, angelegt, so dass die Öffnung 321 mit dem Innenraum 304 des Schlauchabschnitts 303 kommuniziert.

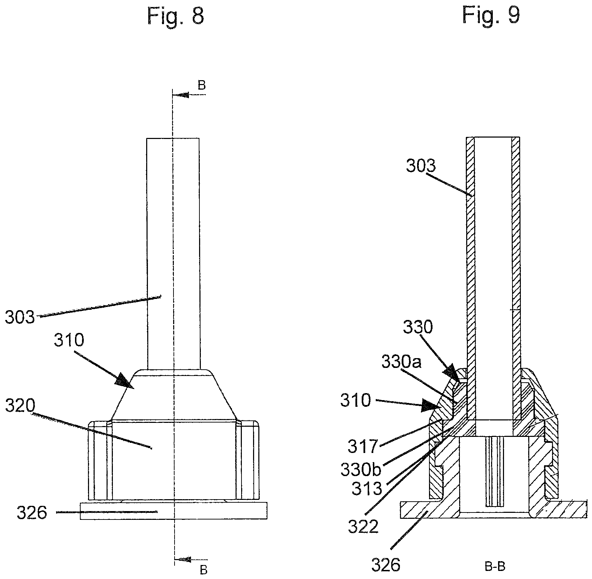

Wie man besonders gut in der Darstellung der Figur 9 erkennt, wird das Zusammenquetschen der ringförmigen Dichtfläche 330b dadurch erreicht, dass der Schlauchadapter 310 mit Hilfselementen 316, der auf dem Schlauchabschnitt 303 aufgefädelt ist, Nuten 313 aufweist, welche in einer ersten Orientierung des Schlauchadapters 310 dessen Aufschieben auf die Verriegelungselemente 327 erlauben und dann schräg verlaufen, so dass beim Rotieren des Schlauchadapters 310 in eine zweite Orientierung die durch eine in Innenraum 310 des umlaufende Stufe gebildete erste Anlagefläche 317 in Richtung auf die zweite Anlagefläche 322 hin gezogen wird.

### Bezugszeichenliste

- 1,101,201,301: Schlauch-Schnellverbinder
- 2,202,302: Absaugschlauch
- 3,203,303: Schlauchabschnitt
- 4,204,304: Innenraum
- 10,110,210,310: Schlauchadapter
- 11,111,211: Abschnitt
- 12,112,212,312: Durchgangsöffnung
- 13,113,213,327: Verriegelungselement
- 14: Ausnehmung
- 15: Stufe
- 16,116,216,316: Hilfselement
- 17,117,217,317: erste Anlagefläche
- 20,120,220,320: Schlauchadapter-Gegenstück
- 21,121,221,321: Öffnung
- 21a,21b,121a,121b: Ausnehmung
- 22,122,222,322: zweite Anlagefläche
- 23,123,223: dritte Anlagefläche
- 24: Vorsprung
- 25: vorspringender Abschnitt
- 30,130,230,330: Dichtung mit mindestens einer ringförmigen Dichtfläche
- 118: Aufsteckkonus
- 226,326: Abschnitt
- 231: Innenkontur
- 313: Nut
- 330a: Halteabschnitt
- 330b: ringförmige Dichtfläche
- d: Abstand
- Dₐ: Durchmesser
- D_{s,außen}: Außendurchmesser
- D_{s,innen}: Innendurchmesser

## Patentansprüche

1. Verfahren zur Verbindung eines Absaugschlauchs (2,202,302) für Körperflüssigkeiten mit einem Behälter mit den Schritten
- Bereitstellen eines an einem Schlauchabschnitt (3,203,303) angeordneten Schlauchadapters (10,110,210, 310), einer Dichtung mit mindestens einer ringförmigen Dichtfläche (30,130,230,330) und eines Behälters, an dem ein Schlauchadapter-Gegenstück (20,120,220,320) angeordnet ist,
- Herstellen eines mechanischen Kontakts zwischen einer ersten Anlagefläche (17,117,217,317), die am Schlauchadapter (10,110,210,310) vorgesehen ist, der Dichtung mit mindestens einer ringförmigen Dichtfläche (30,130,230,330) und einer zweiten Anlagefläche (22,122,222,322), die am Schlauchadapter-Gegenstück (20,120,220,320) vorgesehen ist, in einer ersten Orientierung des Schlauchadapters (10,110,210,310) relativ zum Schlauchadapter-Gegenstück (20,120,220,320), und
- Rotieren des Schlauchadapters (10,110,210,310) aus der ersten Orientierung in eine zweite Orientierung, in der durch eine Wechselwirkung zwischen Abschnitten des Schlauchadapters (10,110,210,310) und Abschnitten des Schlauchadapter-Gegenstücks (20,120,220,320), so dass die Dichtung mit mindestens einer ringförmigen Dichtfläche (30,130,230,330) zwischen der ersten Anlagefläche (17,117, 217,317) und der zweiten Anlagefläche (22,122,222,322) eingequetscht wird,
**dadurch gekennzeichnet, dass** zum Bereitstellen des an dem Schlauchadapter (10,110,210,310) angeordneten Schlauchabschnitts (3,203,303) ein Endabschnitt des Schlauchabschnitts (3,203,303), dessen innerer Querschnitt dem Querschnitt der Durchgangsöffnung (12,112, 212,312) entspricht, in eine sich schlauchseitig an die Durchgangsöffnung (12,112,212,312) anschließende, mit der Durchgangsöffnung (12,112,212,312) konzentrische Ausnehmung (14), deren Ausdehnung an den äußeren Querschnitt des Schlauchabschnitts (3,203,303) angepasst ist, eingeschoben wird bis die Stirnseite des Endabschnitts des Schlauchabschnitts (3,203,303) auf einer Stufe (15) aufliegt und die Außenwand des Endabschnitts des Schlauchabschnitts (3,203,303) an der Wand der Ausnehmung (14) anliegt und mindestens entweder die Stirnseite des Endabschnitts des Schlauchabschnitts (3,203,303) oder die Außenwand des Endabschnitts des Schlauchabschnitts (3,203,303) fixiert wird und dass beim Rotieren des mit dem Teil der mit dem Innenraum (4,204,304) des Schlauchabschnitts (3,203,303) kommunizierenden Durchgangsöffnung (12,112,212,312) versehenen Abschnitts (11, 111,211) des Schlauchadapters (10,110,210,310) aus der ersten Orientierung in eine zweite Orientierung das Verriegelungselement (13,113,213,327) zumindest während Teilen der Rotation entlang einer dritten Anlagefläche (23,123,223) geführt wird, deren Abstand (d) zur zweiten Anlagefläche (22,122,222,322) ausgehend von der Position des Verriegelungselements (13,113,213,327) in der ersten Orientierung hin zur Position des Verriegelungselements (13,113,213,327) in der zweiten Orientierung wächst.

2. Verfahren nach Anspruch 1,
**dadurch gekennzeichnet, dass** beim Herstellen des mechanischen Kontakts ein Abschnitt des Schlauchadapters (10,110,210,310), in dem zumindest ein Teil einer Durchgangsöffnung (12,112,212,312), die mit dem Innenraum (4,204,304) des Schlauchabschnitts (3,203,303) kommuniziert, verläuft, und an dem mindestens ein Verriegelungselement (13,113,213,327) angeordnet ist, in der ersten Orientierung in eine das Schlauchadapter-Gegenstück (20,120,220,320) durchsetzende Öffnung (21,121,221,321) eingeführt wird bis das Verriegelungselement (13,113,213, 327) und ein Zugang zur Durchgangsöffnung (12,112,212,312) auf der anderen Seite des Schlauchadapter-Gegenstücks (20,120,220,320) aus der das Schlauchadapter-Gegenstück (20,120,220,320) durchsetzenden Öffnung (21,121,221,321) austreten,
wobei während des Einführens des mit dem Teil der mit dem Innenraum (4,204,304) des Schlauchabschnitts (3,203,303) kommunizierenden Durchgangsöffnung (12,112,212,312) versehenen Abschnitts (11,111,211) des Schlauchadapters (10, 110,210,310) die Dichtung mit mindestens einer ringförmigen Dichtfläche (30,130,230,330) auf dem mit Teil der mit dem Innenraum (4,204,304) des Schlauchabschnitts (3,203,303) kommunizierenden Durchgangsöffnung (12,112, 212,312) versehenen Abschnitt (11,111,211) des Schlauchadapters (10,110,210,310) aufgesteckt und im Raumbereich zwischen einer ersten Anlagefläche (17,117,217,317), die am Schlauchadapter (10,110,210,310) vorgesehen ist und einer zweiten Anlagefläche (22,122,222,322), die am Schlauchadapter-Gegenstück (20,120,220,320) vorgesehen ist angeordnet ist.

3. Verfahren nach Anspruch 2,
**dadurch gekennzeichnet, dass** beim Rotieren des Schlauchadapters (10,110,210,310) aus der ersten Orientierung in eine zweite Orientierung das Verriegelungselement (13,113,213,327) in eine Position gebracht wird, in der es das Schlauchadapter-Gegenstück (20,120, 220,320) in Richtung auf den Schlauchabschnitt (3,203,303) hin drückt, so dass die Dichtung mit mindestens einer ringförmigen Dichtfläche (30,130,230,330) zwischen der ersten Anlagefläche (17,117,217,317) und der zweiten Anlagefläche (22,122,222,322) eingequetscht wird.

4. Verfahren nach einem der Ansprüche 1 bis 3,
**dadurch gekennzeichnet, dass** beim Rotieren des mit dem Teil der mit dem Innenraum (4,204,304) des Schlauchabschnitts (3,203,303) kommunizierenden Durchgangsöffnung (12,112,212,312) versehenen Abschnitts (11, 111,211) des Schlauchadapters (10,110,210,310) aus der ersten Orientierung in eine zweite Orientierung das Verriegelungselement (13,113,213,313) vor dem Erreichen der zweiten Orientierung eine Position durchläuft, in der die Dichtung mit mindestens einer ringförmigen Dichtfläche (30,130,230,330) stärker zusammengequetscht ist als in der zweiten Orientierung.

5. Schlauch-Schnellverbinder (1,101,201,301) mit einem Schlauchadapter (10,110,210,310), mit einer Dichtung mit mindestens einer ringförmigen Dichtfläche (30,130,230,330) und mit einem Schlauchadapter-Gegenstück (20,120,220,320), wobei der Schlauchadapter (10,110,210,310) auf seiner bestimmungsgemäß dem Schlauch zugewandten Seite einen Schlauchanschluss zur Verbindung mit einem Schlauchabschnitt (3,203,303) aufweist und auf der bestimmungsgemäß dem Schlauchadapter-Gegenstück (20,120,220,320) zugewandten Seite eine erste Anlagefläche (17,117,217,317) für die Dichtung mit mindestens einer ringförmigen Dichtfläche (30,130,230,330) aufweist,
wobei das Schlauchadapter-Gegenstück (20,120,220,320) eine zweite Anlagefläche (22,122,222,322) für die Dichtung mit mindestens einer ringförmigen Dichtfläche (30,130,230,330) auf seiner bestimmungsgemäß dem Schlauchadapter (10,110, 210,310) zugewandten Seite aufweist, und
wobei Schlauchadapter (10,110,210,310) und Schlauchadapter-Gegenstück (20,120,220,320) jeweils Mittel aufweisen, die derart miteinander wechselwirken, dass durch Rotation des Schlauchadapters (10,110,210,310) relativ zum Schlauchadapter-Gegenstück (20,120,220,320) von einer ersten Orientierung zu einer zweiten Orientierung, die Dichtung mit mindestens einer ringförmigen Dichtfläche (30, 130,230,330) zwischen der ersten Anlagefläche (17,117,217, 317) und der zweiten Anlagefläche (22,122,222,322) eingequetscht wird,
**dadurch gekennzeichnet, dass** an dem Schlauchadapter-Gegenstück (20,120,220,320) eine dritte Anlagefläche (23,123,223) vorhanden ist, deren Abstand (d) zur zweiten Anlagefläche (22,122,222,322) von der Position des Verriegelungselements (13,113,213,327) ausgehend von der ersten Orientierung hin zur Position des Verriegelungselements (13,113,213,327) in der zweiten Orientierung zumindest abschnittsweise wächst und dass beim Rotieren des mit dem mit dem Innenraum des Schlauchabschnitts (3, 203,303) kommunizierenden Durchgangsöffnung (12,112,212, 312) versehenen Abschnitts (11,111,211,311) des Schlauchadapters (10,110,210,310) aus der ersten Orientierung in die zweite Orientierung das Verriegelungselement (13,113, 213,313) zumindest abschnittsweise entlang dieser dritten Anlagefläche (23,123,223) geführt wird.

6. Schlauch-Schnellverbinder (1,101,201,301) nach Anspruch 5, **dadurch gekennzeichnet, dass** das Schlauchadapter-Gegenstück von einer Öffnung (21,121,221, 321) zur Aufnahme eines Abschnitts des Schlauchadapters (10,110,210,310) in mindestens einer Orientierung des Schlauchadapters (10,110,210,310) durchsetzt wird,
dass der Schlauchadapter (10,110,210,310) eine Durchgangsöffnung (12,112,212,312), die mit dem Innenraum (4,204, 304) des Schlauchabschnitts (3,203,303) kommuniziert, und einen Abschnitt (11,11,211) zum Hindurchführen durch die Öffnung (21,121,221) im Schlauchadapter-Gegenstück (20, 120,220,320) zur Aufnahme eines Abschnitts (11,111,211) des Schlauchadapters (10,110,210,310) in mindestens einer Orientierung des Schlauchadapters (10,110,210,310) aufweist, an dem mindestens ein Verriegelungselement (13,113, 213,327) angeordnet ist und in dem zumindest ein Teil der Durchgangsöffnung (12,112,212,312), die mit dem Innenraum (4,204,304) des Schlauchabschnitts (3,203,303) kommuniziert, verläuft, und
dass die Öffnung (21,121,221,321) zur Aufnahme eines Abschnitts (11,111,211) des Schlauchadapters (10,110,210, 310) in mindestens einer Orientierung des Schlauchadapters (10,110,210,310) und der Abschnitt (11,111,211) des Schlauchadapters (10,110,210,310) zum Hindurchführen durch die Öffnung (21,121,221,321) zur Aufnahme des Abschnitts (11,111,211) des Schlauchadapters (10,110,210,310) derart aufeinander abgestimmt sind, dass das Verriegelungselement (13,113,213,327) durch Rotation des Schlauchadapters (10, 110,210,310) aus einer ersten Orientierung, in der es durch die Öffnung (21,121,221,321) zur Aufnahme eines Abschnitts des Schlauchadapters (10,110,210,310) hindurchführbar ist, in eine zweite Orientierung, in der das Verriegelungselement (13,113,213,327) das Schlauchadapter-Gegenstück (20,120,220,320) in Richtung auf den Schlauchabschnitt (3,203,303) hin drückt, überführbar ist.

7. Schlauch-Schnellverbinder (1,101,201,301) nach Anspruch 6, **dadurch gekennzeichnet, dass** der innere Querschnitt des Endabschnitts des Schlauchabschnitts (3,203,303) dem Querschnitt der Durchgangsöffnung (12,112,212,312) entspricht und dass der Endabschnitt des Schlauchabschnitts (3,203,303) in einer sich schlauchseitig an die Durchgangsöffnung (12,112,212,312) anschließenden, mit der Durchgangsöffnung (12,112,212,312) konzentrischen Ausnehmung (14), deren Ausdehnung an den äußeren Querschnitt des Endabschnitts des Schlauchabschnitts (3, 203,303) angepasst ist angeordnet ist, so dass die Stirnseite des Endabschnitts des Schlauchabschnitts (3,203,303) auf einer Stufe (15) aufliegt und die Außenwand des Endabschnitts des Schlauchabschnitts (3,203,303) an der Wand der Ausnehmung (14) anliegt und mindestens entweder die Stirnseite des Endabschnitts des Schlauchabschnitts (3, 203,303) oder die Außenwand des Endabschnitts des Schlauchabschnitts (3,203,303) fixiert ist.

8. Schlauch-Schnellverbinder (1,101,201,301) nach Anspruch 7,
**dadurch gekennzeichnet, dass** die Stirnseite des Endabschnitts des Schlauchabschnitts (3, 203,303) und/oder die Außenwand des Endabschnitts des Schlauchabschnitts (3,203,303) durch Stoffschluss am Schlauchadapter (10,110,210,310) fixiert ist.

9. Schlauch-Schnellverbinder (1,101,201,301) nach einem der Ansprüche 6 bis 8,
**dadurch gekennzeichnet, dass** an dem Schlauchadapter (10,110,210,310) Hilfselemente (16,116, 216,316) zur Unterstützung der Krafteinleitung beim Herbeiführen einer Rotationsbewegung des Schlauchadapters (10,110,210,310) um eine durch die Durchgangsöffnung (12, 112,212,312) definierte Achse herum vorhanden sind.

## Claims

1. Method for connecting a suction hose (2, 202, 302) for bodily fluids to a container, said method having the steps of
- providing a hose adapter (10, 110, 210, 310) arranged on a hose portion (3, 203, 303), a seal having at least one annular sealing surface (30, 130, 230, 330), and a container on which a hose adapter mating part (20, 120, 220, 320) is arranged,
- establishing mechanical contact between a first contact surface (17, 117, 217, 317) provided on the hose adapter (10, 110, 210, 310), the seal comprising at least one annular sealing surface (30, 130, 230, 330), and a second contact surface (22, 122, 222, 322) provided on the hose adapter mating part (20, 120, 220, 320), in a first orientation of the hose adapter (10, 110, 210, 310) relative to the hose adapter mating part (20, 120, 220, 320), and
- rotating the hose adapter (10, 110, 210, 310) from the first orientation into a second orientation in which by interaction between portions of the hose adapter (10, 110, 210, 310) and portions of the hose adapter mating part (20, 120, 220, 320), such that the seal comprising the at least one annular sealing surface (30, 130, 230, 330) is squeezed between the first contact surface (17, 117, 217, 317) and the second contact surface (22, 122, 222, 322), **characterised in that** for providing the hose portion (3, 203, 303) arranged on the hose adapter (10, 110, 210, 310) an end portion of the hose portion (3, 203, 303), the inner cross-section of which corresponds to the cross-section of the through-opening (12, 122, 212, 312), is pushed into a recess (14) which adjoins the through-opening (12, 112, 212, 312) on the hose side and which is concentric with the through-opening (12, 112, 212, 312) and the extension of which is matched to the outer cross-section of the hose portion (3, 203, 303), until the end face of the end portion of the hose portion (3, 203, 303) rests on a step (15) and the outside wall of the end portion of the hose portion (3, 203, 303) rests on the wall of the recess (14), and at least either the end face of the end portion of the hose portion (3, 203, 303) or the outside wall of the end portion of the hose portion (3, 203, 303) is fixed, and **in that** upon rotation of the portion (11, 111, 211) of the hose adapter (10, 110, 210, 310) provided with the part of the through-opening (12, 112, 212, 312) communicating with the interior (4, 204, 304) of the hose portion (3, 203, 303) out of a first orientation into a first orientation the locking element (13, 113, 213, 327) is guided along a third contact surface (23, 123, 223) at least during parts of the rotation, the spacing (d) of which contact surface from the second contact surface (22, 122, 222, 322) from the position of the locking element (13, 113, 213, 327) in the first orientation increases towards the position of the locking element (13, 113, 213, 327) in the second orientation.

2. Method according to claim 1,
**characterised in that** when establishing the mechanical contact, a portion of the hose adapter (10, 110, 210, 310) in which at least a part of a through-opening (12, 112, 212, 312) communicating with the interior (4, 204, 304) of the hose portion (3, 203, 303) extends and on which at least one locking element (13, 113, 213, 327) is arranged is inserted, in the first orientation, into an opening (21, 121, 221, 321) that passes through the hose adapter mating part (20, 120, 220, 320) until the locking element (13, 113, 213, 327) and access to the through-opening (12, 112, 212, 312) on the other side of the hose adapter mating part (20, 120, 220, 320) emerge from the opening (21, 121, 221, 321) passing through the hose adapter mating part (20, 120, 220, 320),
wherein during the insertion of the portion (11, 111, 211) of the hose adapter (10, 110, 210, 310) provided with the part of the through-opening (12, 112, 212, 312) communicating with the interior (4, 204, 304) of the hose portion (3, 203, 303) the seal comprising the at least one annular sealing surface (30, 130, 230, 330) is plugged onto the portion (11, 111, 211) of the hose adapter (10, 110, 210, 310) provided with part of the through-opening (12, 112, 212, 312) communicating with the interior (4, 204, 304) of the hose portion (3, 203, 303) and is arranged in the spatial region between a first contact surface (17, 117, 217, 317) provided on the hose adapter (10, 110, 210, 310) and a second contact surface (22, 122, 222, 322) provided on the hose adapter mating part (20, 120, 220, 320).

3. Method according to claim 2,
**characterised in that** upon rotation of the hose adapter (10, 110, 210, 310) from the first orientation into a second orientation, the locking element (13, 113, 213, 327) is brought into a position in which it presses the hose adapter mating part (20, 120, 220, 320) in the direction towards the hose portion (3, 203, 303), such that the seal comprising at least one annular sealing surface (30, 130, 230, 330) is squeezed between the first contact surface (17, 117, 217, 317) and the second contact surface (22, 122, 222, 322).

4. Method according to any of claims 1 to 3,
**characterised in that** upon rotation of the portion (11, 111, 211) of the hose adapter (10, 110, 210, 310) provided with the part of the through-opening (12, 112, 212, 312) communicating with the interior (4, 204, 304) of the hose portion (3, 203, 303) from the first orientation into a second orientation, the locking element (13, 113, 213, 313) passes through a position, before reaching the second orientation, in which the seal comprising at least one annular sealing surface (30, 130, 230, 330) is squeezed together more significantly than in the second orientation.

5. Hose quick connector (1, 101, 201, 301) comprising a hose adapter (10, 110, 210, 310) comprising a seal having at least one annular sealing surface (30, 130, 230, 330), and comprising a hose adapter mating part (20, 120, 220, 320), wherein the hose adapter (10, 110, 210, 310) comprises a hose fitting, on the side thereof intended to face the hose, for connection to a hose portion (3, 203, 303), and comprises a first contact surface (17, 117, 217, 317), on the side thereof intended to face the hose adapter mating part (20, 120, 220, 320), for the seal comprising at least one annular sealing surface (30, 130, 230, 330),
wherein the hose adapter mating part (20, 120, 220, 320) comprises a second contact surface (22, 122, 222, 322) for the seal comprising at least one annular sealing surface (30, 130, 230, 330) on the side thereof intended to face the hose adapter (10, 110, 210, 310), and
wherein the hose adapter (10, 110, 210, 310) and hose adapter mating part (20, 120, 220, 320) each comprise means that interact with one another in such a way that the seal comprising at least one annular sealing surface (30, 130, 230, 330) is squeezed between the first contact surface (17, 117, 217, 317) and the second contact surface (22, 122, 222, 322) by rotation of the hose adapter (10, 110, 210, 310) relative to the hose adapter mating part (20, 120, 220, 320) from a first orientation to a second orientation,
**characterised in that** a third contact surface (23, 123, 223) is provided on the hose adapter mating part (20, 120, 220, 320), the spacing (d) of which contact surface from the second contact surface (22, 122, 222, 322) increases at least in portions from the position of the locking element (13, 113, 213, 327) proceeding from the first orientation towards the position of the locking element (13, 113, 213, 327) in the second orientation, and **in that** upon rotation of the portion (11, 111, 211, 311) of the hose adapter (10, 110, 210, 310) provided with the through-opening (12, 112, 212, 312) communicating with the interior of the hose portion (3, 203, 303) from the first orientation into the second orientation the locking element (13, 113, 213, 313) is guided along said third contact surface (23, 123, 223) at least in portions.

6. Hose quick connector (1, 101, 201, 301) according to claim 5,
**characterised in that** the hose adapter mating part is penetrated by an opening (21, 121, 221, 321) for receiving a portion of the hose adapter (10, 110, 210, 310) in at least one orientation of the hose adapter (10, 110, 210, 310),
**in that** the hose adapter comprises a through-opening (12, 112, 212, 312) which communicates with the interior (4, 204, 304) of the hose portion (3, 203, 303), and a portion (11, 11, 211) for guiding through the opening (21, 121, 221) in the hose adapter mating part (20, 120, 220, 320) for receiving a portion (11, 111, 211) of the hose adapter (10, 110, 210, 310) in at least one orientation of the hose adapter (10, 110, 210, 310), on which at least one locking element (13, 113, 213, 327) is arranged and in which at least a part of the through-opening (12, 112, 212, 312), which communicates with the interior (4, 204, 304) of the hoses portion (3, 203, 303), extends, and
**in that** the opening (21, 121, 221, 321) for receiving a portion (11, 111, 211) of the hose adapter (10, 110, 210, 310) in at least one orientation of the hose adapter (10, 110, 210, 310), and the portion (11, 111, 211) of the hose adapter (10, 110, 210, 310) for guiding through the opening (21, 121, 221, 321) for receiving the portion (11, 111, 211) of the hose adapter (10, 110, 210, 310), are matched to one another in such a way that the locking element (13, 113, 213, 327) can be transferred, by rotation of the hose adapter (10, 110, 210, 310), from a first orientation in which it can be guided through the opening (21, 121, 221, 321) for receiving a portion of the hose adapter (10, 110, 210, 310), into a second orientation in which the locking element (13, 113, 213, 327) pushes the hose adapter mating part (20, 120, 220, 320) in the direction towards the hose portion (3, 203, 303).

7. Hose quick connector (1, 101, 201, 301) according to claim 6,
**characterised in that** the inner cross-section of the end portion of the hose portion (3, 203, 303) corresponds to the cross-section of the through-opening (12, 112, 212, 312), and **in that** the end portion of the hose portion (3, 203, 303) is arranged in a recess (14) that adjoins the through-opening (12, 112, 212, 312) on the hose side and is concentric with the through-opening (12, 112, 212, 312) and the extension of which is matched to the outer cross-section of the end portion of the hose portion (3, 203, 303), such that the end face of the end portion of the hose portion (3, 203, 303) rests on a step (15) and the outside wall of the end portion of the hose portion (3, 203, 303) rests on the wall of the recess (14), and at least either the end face of the end portion of the hose portion (3, 203, 303) or the outside wall of the end portion of the hose portion (3, 203, 303) is fixed.

8. Hose quick connector (1, 101, 201, 301) according to claim 7,
**characterised in that** the end face of the end portion of the hose portion (3, 203, 303) and/or the outside wall of the end portion of the hose portion (3, 203, 303) is fixed to the hose adapter (10, 110, 210, 310) by an integral connection.

9. Hose quick connector (1, 101, 201, 301) according to any of claims 6 to 8,
**characterised in that** auxiliary elements (16, 116, 216, 316) for assisting the force introduction when performing the rotational movement of the hose adapter (10, 110, 210, 310) about an axis defined by the through-opening (12, 112, 212, 312) are provided.

## Revendications

1. Procédé de raccordement d'un tuyau (2, 202, 302) souple d'aspiration de liquide corporel à un récipient comprenant les stades
- se procurer un adaptateur (10, 110, 210, 310) de tuyau souple monté sur un tronçon (3, 203, 303) du tuyau souple, une garniture d'étanchéité ayant au moins une surface (30, 130, 230, 330) annulaire d'étanchéité et un récipient, sur lequel est monté une pièce (20, 120, 220, 320) antagoniste d'un adaptateur de tuyau souple,
- ménager un contact mécanique entre une première surface (17, 117, 217, 317) d'application, qui est prévue sur l'adaptateur (10, 110, 210, 310) de tuyau souple, de la garniture d'étanchéité avec au moins une surface (30, 130, 230, 330) annulaire d'étanchéité, et une deuxième surface (22, 122, 222, 322) d'application, qui est prévue sur la pièce (20, 120, 220, 320) antagoniste d'adaptateur de tuyau souple dans une première orientation de l'adaptateur (10, 110, 210, 310) de tuyau souple par rapport à la pièce (20, 120, 220, 320) antagoniste d'adaptateur de tuyau souple et
- faire tourner l'adaptateur (10, 110, 210, 310) de tuyau souple de la première orientation à une deuxième orientation, dans laquelle, par une interaction entre des parties de l'adaptateur (10, 110, 210, 310) de tuyau souple et des parties de la pièce (20, 120, 220, 320) antagoniste d'adaptateur de tuyau souple, de manière à ce que la garniture d'étanchéité avec au moins une surface (30, 130, 230, 330) annulaire d'étanchéité soit serrée entre la première surface (17, 117, 217, 317) d'application et la deuxième surface (22, 122, 222, 322) d'application,
**caractérisé en ce que**, pour se procurer le tronçon (3, 203, 303) de tuyau souple, monté sur l'adaptateur (10, 110, 210, 310) de tuyau souple on fait glisser un tronçon d'extrémité du tronçon (3, 203, 303) de tuyau souple, dont la section transversale intérieure correspond à la section transversale de l'ouverture (12, 112, 212, 312) de passage, dans un évidement (14) se raccordant du côté du tuyau souple à l'ouverture (12, 112, 212, 312) de passage et concentrique à l'ouverture (12, 112, 212, 312) de passage, dont l'étendue est adaptée à la section transversale extérieure du tronçon (3, 203, 303) de tuyau souple, jusqu'à ce que le côté frontal du tronçon d'extrémité du tronçon (3, 203, 303) de tuyau souple s'applique à un palier (15) et jusqu'à ce que la paroi extérieure du tronçon d'extrémité du tronçon (3, 203, 303) de tuyau souple s'applique à la paroi de l'évidement (14) et au moins ou bien le côté frontal du tronçon d'extrémité du tronçon (3, 203, 303) de tuyau souple ou bien la paroi extérieure du tronçon d'extrémité du tronçon (3, 203, 303) de tuyau souple soit immobilisée et **en ce que**, lors de la rotation de la partie (11, 111, 211) de l'adaptateur (10, 110, 210, 310) de tuyau souple, pourvue de la partie de l'ouverture (12, 112, 212, 312) de passage communiquant avec l'espace (4, 204, 304) intérieur du tronçon (3, 203, 303) de tuyau souple de la première orientation à une deuxième orientation, on guide l'élément (13, 113, 213, 327) de verrouillage au moins pendant des parties de la rotation le long d'une troisième surface (23, 123, 223) d'application, dont la distance (d) à la deuxième surface (22, 122, 222, 322) d'application augmente à partir de la position de l'élément (13, 113, 213, 327) de verrouillage dans la première orientation jusqu'à la position de l'élément (13, 113, 213, 327) de verrouillage dans la deuxième orientation.

2. Procédé suivant la revendication 1,
**caractérisé en ce que**, lorsque l'on ménage le contact mécanique une partie de l'adaptateur (10, 110, 210, 310) de tuyau souple, dans laquelle s'étend au moins une partie d'une ouverture (12, 112, 212, 312) de passage, qui communique avec l'espace (4, 204, 304) intérieur du tronçon (3, 203, 303) de tuyau souple et sur laquelle est disposée au moins un élément (13, 113, 213, 327) de verrouillage, est introduite dans la première orientation dans une ouverture (21, 121, 221, 321) traversant la pièce (20, 120, 220, 320) antagoniste d'adaptateur de tuyau souple jusqu'à ce que l'élément (13, 113, 213, 327) de verrouillage et un accès à l'ouverture (12, 112, 212, 312) de passage de l'autre côté de la pièce (20, 120, 220, 320) antagoniste d'adaptateur de tuyau souple sortent de l'ouverture (21, 121, 221, 321) traversant la pièce (20, 120, 220, 320) antagoniste d'adaptateur de tuyau souple,
dans lequel, pendant l'introduction du tronçon (11, 111, 211) de l'adaptateur (10, 110, 210, 310) de tuyau souple, pourvu de la partie de l'ouverture (12, 112, 212, 312) de passage communiquant avec l'espace (4, 204, 304) intérieur du tronçon (3, 203, 303) de tuyau souple, la garniture d'étanchéité avec au moins une surface (30, 130, 230, 330) annulaire d'étanchéité est enfilée sur le tronçon (11, 111, 211) de l'adaptateur (10, 110, 210, 310) de tuyau souple, pourvu d'une partie de l'ouverture (12, 112, 212, 312) de passage communiquant avec l'espace (4, 204, 304) intérieur du tronçon (3, 203, 303) de tuyau souple, et est disposée dans la partie de l'espace comprise entre une première surface (17, 117, 217, 317) d'application qui est prévue sur l'adaptateur (10, 110, 210, 310) de tuyau souple et une deuxième surface (22, 122, 222, 322) d'application qui est prévue sur la pièce (20, 120, 220, 320) antagoniste de l'adaptateur de tuyau souple.

3. Procédé suivant la revendication 2,
**caractérisé en ce que** lorsque l'adaptateur (10, 110, 210, 310) de tuyau souple passe de la première orientation à une deuxième orientation on met l'élément (13, 113, 213, 327) de verrouillage dans une position dans laquelle il pousse la pièce (20, 120, 220, 320) antagoniste de l'adaptateur de tuyau souple en direction du tronçon (3, 203, 303) de tuyau souple de sorte que la garniture d'étanchéité avec au moins une surface (30, 130, 230, 330) annulaire d'étanchéité est serrée entre la première surface (17, 117, 217, 317) d'application et la deuxième surface (22, 122, 222, 322) d'application.

4. Procédé suivant l'une des revendications 1 à 3,
**caractérisé en ce que** lorsque le tronçon (11, 111, 211) de l'adaptateur (10, 110, 210, 310) de tuyau souple passe, pourvu de la partie de l'ouverture (12, 112, 212, 312) de passage communiquant avec l'espace (4, 204, 304) intérieur du tronçon (3, 203, 303) de tuyau souple, de la première orientation à une deuxième orientation l'élément (13, 113, 213, 313) de verrouillage passe avant d'atteindre la deuxième orientation dans une position dans laquelle la garniture d'étanchéité est par au moins une surface (30, 130, 230, 330) annulaire d'étanchéité serrée plus fortement que dans la deuxième orientation.

5. Connecteur (1, 101, 201, 301) rapide de tuyau souple comprenant un adaptateur (10, 110, 210, 310) de tuyau souple comprenant une garniture d'étanchéité avec au moins une surface (30, 130, 230, 330) annulaire d'étanchéité et comprenant une pièce (20, 120, 220, 320) antagoniste d'adaptateur de tuyau souple dans lequel l'adaptateur (10, 110, 210, 310) de tuyau souple a, de son côté tourné vers le tuyau souple conformément aux prescriptions, un raccord de tuyau souple à raccorder à un tronçon (3, 203, 303) de tuyau souple et, du côté tourné vers la pièce (20, 120, 220, 320) antagoniste de l'adaptateur de tuyau souple conformément aux prescriptions, une première surface (17, 117, 217, 317) d'application de la garniture d'étanchéité par au moins une surface (30, 130, 230, 330) annulaire d'étanchéité,
dans lequel la pièce (20, 120, 220, 320) antagoniste de l'adaptateur de tuyau souple a une deuxième surface (22, 122, 222, 322) d'application de la garniture d'étanchéité par au moins une surface (30, 130, 230, 330) annulaire d'étanchéité, de son côté tourné vers l'adaptateur (10, 110, 210, 310) de tuyau souple conformément aux prescriptions, et
dans lequel l'adaptateur (10, 110, 210, 310) de tuyau souple et la pièce (20, 120, 220, 320) antagoniste d'adaptateur de tuyau souple on chacun des moyens qui interagissent entre eux de manière à ce que, par rotation de l'adaptateur (10, 110, 210, 310) de tuyau souple par rapport à la pièce (20, 120, 220, 320) antagoniste de l'adaptateur de tuyau souple d'une première orientation à une deuxième orientation, la garniture soit par au moins une surface (30, 130, 230, 330) annulaire d'étanchéité pressée entre la première surface (17, 117, 217, 317) d'application et la deuxième surface (22, 122, 222, 322) d'application,
**caractérisé en ce qu'**il y a sur la pièce (20, 120, 220, 320) antagoniste de l'adaptateur de tuyau souple une troisième surface (23, 123, 223) d'application dont la distance (d) à la deuxième surface (22, 122, 222, 322) d'application augmente au moins par endroit de la position de l'élément (13, 113, 213, 327) de verrouillage en partant de la première position vers la position de l'élément (13, 113, 213, 327) de verrouillage dans la deuxième orientation et **en ce que**, lors de la rotation du tronçon (11, 111, 211, 311), pourvu de l'ouverture (12, 112, 212, 312) de passage communiquant avec l'espace intérieur du tronçon (3, 203, 303) de tuyau souple, de l'adaptateur (10, 110, 210, 310) de tuyau souple de la première orientation à la deuxième orientation, l'élément (13, 113, 213, 313) de verrouillage est guidé au moins par endroit le long de cette troisième surface (23, 123, 223) d'application.

6. Connecteur (1, 101, 201, 301) rapide de tuyau souple suivant la revendication 5,
**caractérisé en ce que** la pièce antagoniste de l'adaptateur de tuyau souple est traversée par une ouverture (21, 121, 221, 231) de réception d'une partie de l'adaptateur (10, 110, 210, 310) du tuyau souple dans au moins une orientation de l'adaptateur (10, 110, 210, 310) du tuyau souple,
**en ce que** l'adaptateur (10, 110, 210, 310) du tuyau souple a une ouverture (12, 112, 212, 312) de passage, qui communique avec l'espace (4, 204, 304) intérieur du tronçon (3, 203, 303) de tuyau souple, et une partie (11, 111, 211) pour le passage dans l'ouverture (21, 121, 221, 321) de la pièce (20, 120, 220, 320) antagoniste de l'adaptateur de tuyau souple pour la réception d'une partie (11, 111, 211) de l'adaptateur (10, 110, 210, 310) du tuyau souple dans au moins une orientation de l'adaptateur (10, 110, 210, 310) de tuyau souple, sur lequel est disposé au moins un élément (13, 113, 213, 327) de verrouillage et dans lequel s'étend au moins une partie de l'ouverture (12, 112, 212, 312) de passage, qui communique avec l'espace (4, 204, 304) intérieur du tronçon (3, 203, 303) de tuyau souple, et
**en ce que** l'ouverture (21, 121, 221, 321) de réception d'une partie (11, 111, 211) de l'adaptateur (10, 110, 210, 310) du tuyau souple dans au moins une orientation de l'adaptateur (10, 110, 210, 310) du tuyau souple et la partie (11, 111, 211) de l'adaptateur (10, 110, 210, 310) du tuyau souple sont d'adaptées l'une à l'autre pour le passage par l'ouverture (21, 121, 221, 321) de réception de la partie (11, 111, 211) de l'adaptateur (10, 110, 210, 310) du tuyau souple, de manière à ce que l'élément (13, 113, 213, 327) de verrouillage puisse, par une rotation de l'adaptateur (10, 110, 210, 310) du tuyau souple, passer d'une première orientation, dans laquelle il peut passer dans l'ouverture (21, 121, 221, 321) de réception d'une partie de l'adaptateur (10, 110, 210, 310) du tuyau souple, à une deuxième orientation, dans laquelle l'élément (13, 113, 213, 327) de verrouillage pousse la pièce (20, 120, 220, 320) antagoniste de l'adaptateur de tuyau souple en direction du tronçon (3, 203, 303) de tuyau souple.

7. Connecteur (1, 101, 201, 301) rapide de tuyau souple suivant la revendication 6,
**caractérisé en ce que** la section transversale intérieure du tronçon d'extrémité du tronçon (3, 203, 303) de tuyau souple correspond à la section transversale de l'ouverture (12, 112, 212, 312) de passage et **en ce que** le tronçon d'extrémité du tronçon (3, 203, 303) de tuyau souple est disposé dans un évidement (14) concentrique avec l'ouverture (12, 112, 212, 312) de passage, se raccordant du côté du tuyau souple à l'ouverture (12, 112, 212, 312) de passage et dont l'étendue est adaptée à la section transversale extérieure du tronçon d'extrémité du tronçon (3, 203, 303) du tuyau souple, de sorte que le côté frontal du tronçon d'extrémité du tronçon (3, 203, 303) du tuyau souple s'applique à un palier (15) et que la paroi extérieure du tronçon d'extrémité du tronçon (3, 203, 303) du tuyau s'applique à la paroi d'évidement (14) et au moins ou bien le côté frontal du tronçon d'extrémité du tronçon (3, 203, 303) du tuyau souple, ou bien la paroi extérieure du tronçon d'extrémité du tronçon (3, 203, 303) du conduit souple est immobilisé.

8. Connecteur (1, 101, 201, 301) rapide de tuyau souple suivant la revendication 7,
**caractérisé en ce que** le côté frontal du tronçon d'extrémité du tronçon (3, 203, 303) du tuyau souple et/ou la paroi extérieure du tronçon d'extrémité du tronçon (3, 203, 303) du tuyau souple est fixé à l'adaptateur (10, 110, 210, 310) du tuyau souple par coopération de matière.

9. Connecteur (1, 101, 201, 301) rapide de tuyau souple suivant l'une des revendications 6 à 8,
**caractérisé en ce qu'**il y a sur l'adaptateur (10, 110, 210, 310) du tuyau souple des éléments (16, 116, 216, 316) auxiliaires pour faciliter l'application d'une force lorsqu'un mouvement de rotation de l'adaptateur (10, 110, 210, 310) du tuyau souple autour d'un axe défini par l'ouverture (12, 112, 212, 312) de passage est provoqué.
